# EUROPEAN PATENT SPECIFICATION

(11) **EP 1 658 831 B1**
(45) Date of publication and mention of the grant of the patent: **02.01.2008**
(21) Application number: 04257256.0
(22) Date of filing: 23.11.2004
(51) Int. Cl.: A61F 5/455, A61F 5/441

(54) **Urine collection apparatus**
Apparat zum Sammeln von Urin
Appareil collecteur d'urine

(43) Date of publication of application: 24.05.2006
(73) Proprietor: Onkologie Internasionale Beleggings (Proprietary) Limited, Stellenbosch, Cape Province (ZA)
(72) Inventor: Vermaak, Jan Christiaan, Somerset West, Cape Province (ZA)
(74) Representative: Jones, Helen M.M.

(56) References cited:
- EP-A- 0 348 071
- GB-A- 996 370
- US-A- 4 568 339
- US-A- 4 784 654

## Description

### BACKGROUND OF THE INVENTION

THIS invention relates to urine collection apparatus. The closest prior art is US-A-4 784 654, which defines the preambles of claims 1 and 7.

When a patient is bedridden and/or incontinent, urination may be problematic. Depending on the patient's condition, it may be difficult to urinate without assistance.

It would be desirable to provide an apparatus which would assist a bedridden patient to urinate without assistance, at any time.

### SUMMARY OF THE INVENTION

Urine collection apparatus comprising:
a urine collector shaped for location against the perineal region of a patient adjacent the urethra, the urine collector having an inlet for air and an outlet for air and urine; and
a urine receptacle having an inlet for receiving air and urine from the urine collector, and an outlet for air which is connectable to a source of low pressure,
so that air passing through the inlet of the urine collector entrains urine for delivery to the urine receptacle.

The urine collector is shaped to define a venturi between the inlet and the outlet thereof, the venturi being locatable directly adjacent the patient's urethra when the urine collector is correctly located.

The urine collector may be shaped for use by a female patient.

The urine collector may comprise a hollow body having opposed ends in which the inlet and the outlet are formed, the urine collector having first and second end regions and a central region between the end regions having a cross sectional area less than that of the end regions.

The urine collector is preferably formed from a flexible plastics material.

The apparatus preferably includes a source of low pressure in the form of an impeller or pump arranged to apply suction to the outlet of the urine collector when the inlet of the urine receptacle is connected to the outlet of the urine collector, thereby to cause an airflow through the urine collector.

The source of low pressure may comprise an impeller arranged to be driven by an electric motor and located in a housing, the urine receptacle being locatable in an opening defined in the housing so that the outlet of the urine receptacle is in communication with an air inlet of the impeller.

The invention extends to a urine collector suitable for use with the above defined apparatus, the urine collector being shaped for location against the perineal region of a patient adjacent the urethra and comprising a hollow body defining an inlet for air and an outlet for air and urine.

The body is shaped to define a venturi between the inlet and the outlet thereof, the venturi being locatable directly adjacent the patient's urethra when the urine collector is correctly located.

The body preferably has opposed ends in which the inlet and the outlet are formed, the urine collector having first and second end regions and a central region between the end regions having a cross sectional area less than that of the end regions.

The body may be formed from a flexible plastics material.

### BRIEF DESCRIPTION OF THE DRAWINGS

- Figure 1: is a pictorial view of a urine collector forming part of urine collection apparatus according to the invention;
- Figure 2: is a schematic side view of a urine receptacle and low pressure source forming part of the apparatus of the invention; and
- Figure 3: is a sectional side view of an auxiliary urine sampler of the apparatus.

### DESCRIPTION OF EMBODIMENTS

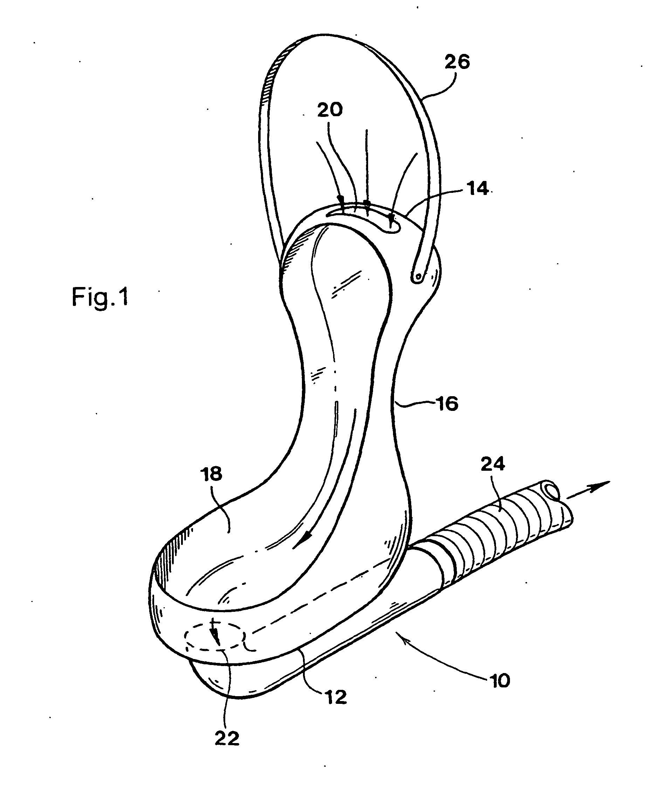

Figure 1 shows a urine collector of the invention which comprises a hollow body 10 having enlarged opposed ends 12 and 14 and a narrowed central region 16. The ends are smoothly rounded. The collector is curved as shown and defines an opening 18 on its inner side which is shaped, together with the curvature of the body of the collector, to fit snugly against the perineal region of a female patient. In order to accommodate different patients, the collector body can conveniently be moulded from a flexible plastics material, and should ideally be disposable.

An air inlet 20 is formed in the first end 14 of the collector, and an outlet 22 for air and urine, which can be connected to a flexible hose or pipe 24, is formed at the other end 12. When the collector is placed against the body of a patient, air flowing into the inlet 20 and out of the outlet 22 entrains urine and carries it away for separation from the air.

In order to enhance the efficiency of urine collection, the central region 16 of the collector is narrowed relative to the ends 12 and 14, effectively defining a venturi in the central region of the collector which is located directly adjacent the patient's urethra in use. The venturi results in an accelerated airflow directly adjacent the urethra, enhancing the efficiency of urine collection.

An elasticated strap 26 is fitted to the end 14 of the collector, which will be uppermost in use, to facilitate attachment of the collector to the patient.

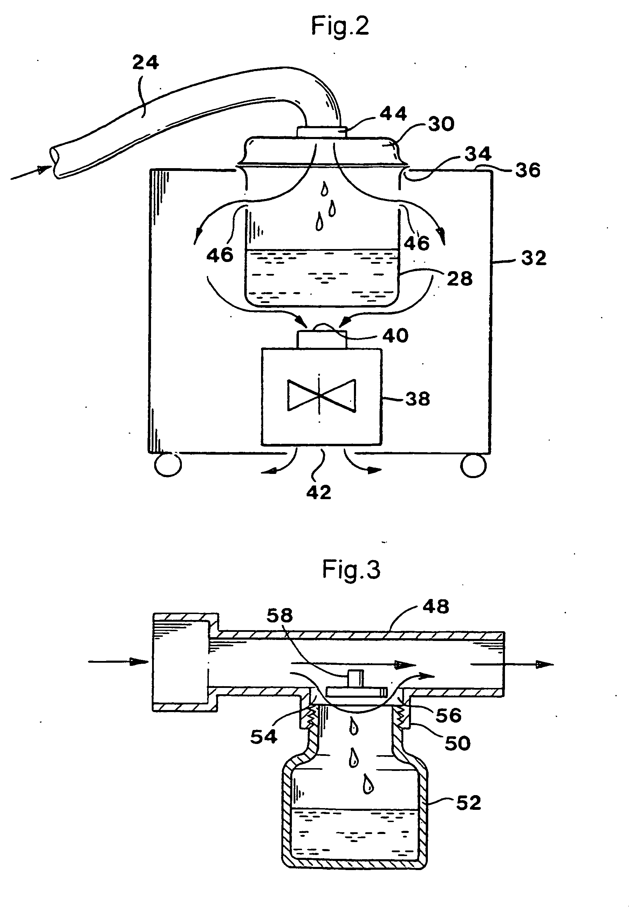

Referring now to Figure 2, a urine receptacle comprising a container 28 with a removable lid 30 is shown received in a housing 32. A circular aperture 34 is formed in the upper surface 36 of the housing for this purpose, with the container ideally having an outwardly extending lip to support it in the aperture.

Also located within the housing is a suction source 38 in the form of an impeller arranged to be driven by an electrical motor, and having an air inlet 40 within the housing and an air outlet 42 at the base of the housing. In the prototype apparatus of the invention, the motor/impeller unit from a domestic vacuum cleaner was used.

The container 28 has an inlet 44 for air and urine in the lid 30 thereof and a pair of air outlets 46 formed in the side of the container near the upper rim thereof.

The flexible hose or pipe 24 is connected between the outlet 22 of the urine collector and the inlet 44 of the container as indicated, and the suction source 38 is operated to cause a flow of air through the apparatus as indicated by the arrows in Figures 1 and 2. Air with entrained urine is drawn through the hose 24 and the urine drops into the container 28, while the air is drawn through the apertures 46 in the container and exhausted from the bottom of the housing 32. When the container 28 is full, it can simply be lifted out of the housing 32 to be emptied or replaced.

Figure 3 shows a urine sampling device which can be used in conjunction with the above described apparatus. It is frequently necessary to take urine samples from patients, and the sampling device was designed to work in conjunction with the above described apparatus to simplify the process.

The sampling device essentially comprises a length of rigid tubing 48 to which sections of hose 24 can be connected. Typically, the sampling device will be connected between the collector 10 and the suction source. The pipe 48 defines a threaded branch 50 into which the neck of a sample bottle 52 can be screwed as indicated. Apertures 54 and 56 are formed in the wall of the pipe 48 in communication with the bore of the branch, and an obstruction in the form of a finger or partition 58 in the pipe between the apertures 54 and 56 causes a portion of the air flowing through the pipe to be diverted through the apertures 54 and 56 as indicated, thereby delivering entrained urine into the container 52.

It will be appreciated that a number of variations of the above described embodiment are possible. For example, the source of low pressure need not be a motor/impeller but could be a steam vacuum line as used in hospitals, or could even be an arrangement making use of a pressure differential such as exists between the interior of an aircraft cabin and the outside atmosphere.

The exact shape and design of the urine collector can also be varied. In practice it was found that it was not essential to maintain an airtight seal between the collector and the body of the patient, and that a degree of air leakage did not impair the functioning of the apparatus.

## Claims

1. Urine collection apparatus comprising:
a urine collector shaped for location against the perineal region of a patient adjacent the urethra, the urine collector having an inlet (20) for air and an outlet (22) for air and urine; and
a urine receptacle having an inlet (44) for receiving air and urine from the urine collector, and an outlet (46) for air which is connectable to a source (38) of low pressure,
**characterised in that** the urine collector is shaped to define a venturi between the inlet (20) and the outlet (22) thereof, the venturi being locatable adjacent the patient's urethra when the urine collector is correctly located, so that air passing through the inlet (20) of the urine collector entrains urine for delivery to the urine receptacle.

2. Urine collection apparatus according to claim 1, wherein the urine collector is shaped for use by a female patient.

3. Urine collection apparatus according to claim 1 or claim 2, wherein the urine collector comprises a hollow body (10) having opposed ends (14, 12) in which the inlet (20) and the outlet (22) are formed, the urine collector having first and second end regions and a central region (16) between the end regions having a cross sectional area less than that of the end regions.

4. Urine collection apparatus according to any one of claims 1 to 3, wherein the urine collector is formed from a flexible plastics material.

5. Urine collection apparatus according to any one of claims 1 to 4, wherein the apparatus includes a source (38) of low pressure in the form of an impeller or pump arranged to apply suction to the outlet (22) of the urine collector when the inlet (44) of the urine receptacle is connected to the outlet (22) of the urine collector, thereby to cause an airflow through the urine collector.

6. Urine collection apparatus according to claim 5, wherein the source (38) of low pressure comprises an impeller arranged to be driven by an electric motor and located in a housing, the urine receptacle being locatable in an opening defined in the housing so that the outlet of the urine receptacle is in communication with an air inlet of the impeller.

7. A urine collector suitable for use with the apparatus of any one of claims 1 to 6, the urine collector being shaped for location against the perineal region of a patient adjacent the urethra and comprising a hollow body (10) defining an inlet (20) for air and an outlet (22) for air and urine, **characterised in that** the body is shaped to define a venturi between the inlet (20) and the outlet (22) thereof, the venturi being locatable directly adjacent the patient's urethra when the urine collector is correctly located.

8. A urine collector according to claim 7, wherein the body has opposed ends (14, 12) in which the inlet (20) and the outlet (22) are formed, the urine collector having first and second end regions and a central region (16) between the end regions having a cross sectional area less than that of the end regions.

9. A urine collector according to claim 7 or claim 8, wherein the body is formed from a flexible plastics material.

## Patentansprüche

1. Vorrichtung zum Sammeln von Urin mit:
einem Urinsammler, der zum Platzieren gegen den perinealen Bereich eines Patienten, angrenzend an die Harnröhre geformt ist, wobei der Urinsammler einen Einlass (20) für Luft und einen Auslass (22) für Luft und Urin aufweist, und
einem Urinbehälter mit einem Einlass (44) zum Aufnehmen von Luft und Urin aus dem Urinkollektor und einem Auslass (46) für Luft, der an eine Unterdruckquelle (38) anschließbar ist,
**dadurch gekennzeichnet, dass** der Urinkollektor so geformt ist, dass er zwischen seinem Einlass (20) und seinem Auslass (22) ein Venturi-Rohr bildet, wobei das Venturi-Rohr angrenzend an die Harnröhre des Patienten platzierbar ist, wenn der Urinsammler bestimmungsgemäß platziert ist, so dass durch den Einlass (20) des Urinsammlers strömende Luft Urin zur Überführung in den Urinbehälter mitreißt.

2. Vorrichtung zum Sammeln von Urin nach Anspruch 1, wobei der Urinsammler für die Verwendung durch einen weiblichen Patienten geformt ist.

3. Vorrichtung zum Sammeln von Urin nach Anspruch 1 oder Anspruch 2, wobei der Urinsammler einen hohlen Korpus (10) mit einander gegenüberliegenden Enden (14, 12), in denen der Einlass (20) und der Auslass (22) gebildet sind, aufweist, wobei der Urinsammler einen ersten und einen zweiten Endbereich und einen mittleren Bereich (16) zwischen den Endbereichen aufweist, der eine Querschnittsfläche aufweist, die kleiner ist als diejenige der Endbereiche.

4. Vorrichtung zum Sammeln von Urin nach irgendeinem der Ansprüche 1 bis 3, wobei der Urinsammler aus einem flexiblen Kunststoffmaterial gebildet ist.

5. Vorrichtung zum Sammeln von Urin nach irgendeinem der Ansprüche 1 bis 4, wobei die Vorrichtung eine Unterdruckquelle (38) in Gestalt eines Flügelrades oder einer Pumpe aufweist, die so angeordnet ist, dass sie auf den Auslass (22) des Urinsammlers eine Saugwirkung ausübt, wenn der Einlass (44) des Urinbehälters mit dem Auslass (22) des Urinsammlers verbunden ist, um so einen Luftstrom durch den Urinsammler zu bewirken.

6. Vorrichtung zum Sammeln von Urin nach Anspruch 5, wobei die Unterdruckquelle (38) ein Flügelrad enthält, das so angeordnet ist, dass es von einem Elektromotor angetrieben wird, und in einem Gehäuse liegt, wobei der Urinbehälter in einer in dem Gehäuse gebildeten Öffnung platzierbar ist, so dass der Auslass des Urinbehälters mit einem Lufteinlass des Flügelrades in Verbindung steht.

7. Für die Verwendung mit der Vorrichtung nach irgendeinem der Ansprüche 1 bis 6 geeigneter Urinsammler, wobei der Urinsammler zum Platzieren gegen den perinealen Bereich eines Patienten, angrenzend an die Harnröhre geformt ist und einen hohlen Korpus (10) aufweist, der einen Einlass (20) für Luft und einen Auslass (22) für Luft und Urin bestimmt, **dadurch gekennzeichnet, dass** der Korpus so geformt ist, dass er zwischen seinem Einlass (20) und seinem Auslass (22) ein Venturi-Rohr bildet, wobei das Venturi-Rohr direkt angrenzend an die Harnröhre des Patienten platzierbar ist, wenn der Urinsammler bestimmungsgemäß platziert ist.

8. Urinsammler nach Anspruch 7, wobei der Korpus einander gegenüberliegenden Enden (14, 12) aufweist, in denen der Einlass (20) und der Auslass (22) gebildet sind, wobei der Urinsammler einen ersten und einen zweiten Endbereich und einen mittleren Bereich (16) zwischen den Endbereichen aufweist, der eine Querschnittsfläche aufweist, die kleiner ist als diejenige der Endbereiche.

9. Urinsammler nach Anspruch 7 oder Anspruch 8, wobei der Korpus aus einem flexiblen Kunststoffmaterial gebildet ist.

## Revendications

1. Appareil collecteur d'urine, comprenant :
- un collecteur d'urine formé pour être placé contre la zone périnéale d'un patient au voisinage de l'urètre, ce collecteur d'urine comportant une entrée (20) pour l'air et une sortie (22) pour l'air et l'urine, et
- un récipient à urine comportant une entrée (44) pour recevoir l'air et l'urine provenant du collecteur d'urine, et une sortie d'air (46) pouvant être connecté à une source (38) de basse pression,
**caractérisé en ce que**
le collecteur d'urine est formé pour définir un venturi entre son entrée (20) et sa sortie (22), le venturi pouvant être placé au voisinage de l'urètre du patient lorsque le collecteur d'urine est correctement placé, de façon que l'air passant par l'entrée (20) du collecteur d'urine entraîne l'urine pour la fournir au récipient à urine.

2. Appareil collecteur d'urine selon la revendication 1,
dans lequel
le collecteur d'urine est formé pour être utilisé par une patiente.

3. Appareil collecteur d'urine selon l'une quelconque des revendications 1 ou 2,
dans lequel
le collecteur d'urine comprend un corps creux (10) muni d'extrémités opposées (14, 12) dans lesquelles sont formées l'entrée (20) et la sortie (22), ce collecteur d'urine comportant une première zone d'extrémité, une seconde zone d'extrémité et une zone centrale (16) entre les zones d'extrémités, cette zone centrale ayant une surface de section transversale inférieure à celle des zones d'extrémités.

4. Appareil collecteur d'urine selon l'une quelconque des revendications 1 à 3,
dans lequel
le collecteur d'urine est formé d'une matière plastique souple.

5. Appareil collecteur d'urine selon l'une quelconque des revendications 1 à 4,
dans lequel
cet appareil comprend une source de basse pression (38) se présentant sous la forme d'un accélérateur ou d'une pompe servant à appliquer une aspiration à la sortie (22) du collecteur d'urine lorsque l'entrée (44) du récipient à urine est reliée à la sortie (22) du collecteur d'urine, pour produire ainsi un débit d'air à travers le collecteur d'urine.

6. Appareil collecteur d'urine selon la revendication 5,
dans lequel
la source (38) de basse pression comprend un accélérateur monté pour être entraîné par un moteur électrique et placé dans un boîtier, le récipient à urine pouvant être placé dans une ouverture définie dans le boîtier, de façon que la sortie du récipient à urine soit en communication avec une entrée d'air de l'accélérateur.

7. Collecteur d'urine destiné à être utilisé dans l'appareil selon l'une quelconque des revendications 1 à 6, ce collecteur d'urine étant formé pour être placé contre la zone périnéale d'un patient au voisinage de l'urètre, et comprenant un corps creux (10) définissant une entrée d'air (20) et une sortie d'air et d'urine (22),
**Caractérisé en ce que**
Le corps est formé pour définir un venturi entre son entrée (20) et sa sortie (22), le venturi pouvant être placé directement au voisinage de l'urètre du patient lorsque le collecteur d'urine est correctement placé.

8. Collecteur d'urine selon la revendication 7,
dans lequel
le corps comporte des extrémités opposées (14, 12) dans lesquelles sont formées l'entrée (20) et la sortie (22), le collecteur d'urine comportant une première zone d'extrémité, une seconde zone d'extrémité et une zone centrale (16) entre les zones d'extrémités, cette zone centrale ayant une surface de section transversale inférieure à celle des zones d'extrémités.

9. Collecteur d'urine selon l'une quelconque des revendication 7 ou 8,
dans lequel
le corps est formé d'une matière plastique souple.
